# EUROPEAN PATENT APPLICATION

(11) **EP 4 516 783 A1**
(43) Date of publication of application: **05.03.2025**
(21) Application number: 23796847.4
(22) Date of filing: 27.04.2023
(51) Int. Cl.: C07D 307/48, A61K 31/341, A61P 3/00, A61P 3/04, A23L 33/10

(54) **METHOD FOR PREPARATION OF COMPOUND POSSESSING 5-PENTYLFURFURAL (5-PF) STRUCTURE AND USE THEREOF**

(30) Priority: 29.04.2022 KR 20220053491
(71) Applicant: Fugenbio Co., Ltd., Yongin-si, Gyeonggi-do 16942 (KR)
(72) Inventor: KIM, Yoon Soo, Yongin-si Gyeonggi-do 16942 (KR)
(74) Representative: Dehns
(86) International application number: PCT/KR2023/005788
(87) International publication number: WO 2023/211205

(57) **Abstract**

The present invention relates to a novel 5-PF-amino acid derivative (5-PF-AA); a method for preparing a 5-PF structured compound or a 5-PF-amino acid derivative; and a pharmaceutical composition containing 5-PF structured compound or a 5-PF-amino acid derivative as an active ingredient for inhibiting adipocyte differentiation or treating metabolic disease.

## Description

### Technical Field

The present disclosure relates to a method for preparing a 5-pentylfurfural (5-PF) structural compound or a 5-PF-amino acid derivative (5-PF-AA) and a pharmaceutical composition for inhibiting adipocyte differentiation or treating metabolic diseases comprising the same as an active ingredient.

### Background Art

Metabolic diseases such as diabetes include insulin-dependent diabetes and non-insulin-dependent diabetes, and they differ in the time of onset, nature of the disease, severity, and treatment method. In the case of insulin-dependent diabetes, insulin secretion is absolutely insufficient, and thus repeated and continuous insulin injections are required. In the case of non-insulin-dependent diabetes, treatment methods such as controlling blood sugar levels through diet and exercise therapy or using pharmaceutical drugs (*e.g.*, oral hypoglycemic agents, insulin medicines, *etc.*) are available.

As hypoglycemic agents, sulfonylureas, biguanides, α-glucosidase inhibitors, *etc.* are used. While sulfonylureas act to promote insulin secretion, they can also cause side effects on the blood, gastrointestinal tract, cardiovascular system, kidneys, and skin, and may also lead to hypoglycemia. Biguanides act by inhibiting glucose production in the liver, but cause side effects on the gastrointestinal tract. Additionally, in the case of α-glucosidase inhibitors, they cause side effects such as abdominal bloating, diarrhea, and liver tissue damage.

Not only hypoglycemic agents, but also pharmaceutical drugs (*e.g*., insulin medicines, *etc.*) often cause side effects such as hypoglycemia, allergies, hyperlipidemia, and polyuria. Recently, there has been active research on preventing or treating diabetes using foods or natural products to reduce these side effects (Korean Patent Publication No. 2005-0090867).

Meanwhile, since the compounds extracted from *Ceriporia lacerate* have antibacterial and antifungal effects, they have been used to treat vaginitis, nephritis, edema, *etc.* Among the compounds extracted from *Ceriporia lacerata,* 5-pentyl-2-furaldehyde, 5-(4-pentyl)-2-furaldehyde, and methyl-3-*p*-anisoloxypropionide are known to be nematocidal components. In particular, 5-pentyl-2-furaldehyde was reported to potently inhibit melanin production in B16F10 cells without cytotoxicity (IC₅₀ = 8.4 µg/mL). However, until recently, there have been no cases in which this substance was used as a pharmaceutical composition for the treatment of metabolic diseases.

In addition, with regard to the preparation method of 5-PF, it is known that in the existing synthetic method, 5-hydroxymethyl furfural (HMF) trifluoromethanesulfonate anhydride (triflate) is reacted using the Grignard substitution method at a reaction temperature of -78°C, which is an extremely low temperature environment (WO2014/179156A1), there are no cases where the 5-PF was easily synthesized under milder reaction environments, and also no application cases, such as synthesis of various new derivatives through functionalization, have been reported.

### [Prior Art Document]

### [Patent Document]

(Patent Document 1) KR Patent Publication No. 2005-0090867

### [Non-Patent Document]

(Non-Patent Document 1) J. Microbiol. Biotechnol. (2012), 22(6), 814-817

### Disclosure of Invention

### Technical Problem

Under the circumstances, the present inventors have confirmed an improved method for preparing a compound with a 5-PF structure, and furthermore, have confirmed that derivatives combining 5-PF and amine compounds (*e.g*., 5-PF-amino acid (AA)) exhibited a therapeutic effect on metabolic diseases through inhibition of adipocyte differentiation without side effects, thereby completing the present disclosure.

Therefore, an object of the present disclosure is to provide an improved process for preparing a compound with a 5-PF structure.

Another object of the present disclosure is to provide a novel 5-PF-AA derivative that exhibits a therapeutic effect on metabolic diseases through inhibition of adipocyte differentiation.

Still another object of the present disclosure is to provide a pharmaceutical composition for inhibiting adipocyte differentiation or treating metabolic diseases, comprising 5-PF or a 5-PF-amino acid (AA) derivative; a hydrate or solvate thereof; or a pharmaceutically acceptable salt thereof as an active ingredient.

### Solution to Problem

In order to achieve the above objects, one aspect of the present disclosure provides a method for preparing a compound comprising a 5-pentylfurfural (5-PF) structure of the following Formula 2, comprising (i) a first step of adding butyllithium (BuLi) dissolved in hexane to a compound of the following Formula 1 dissolved in an aprotic organic solvent to prepare a reaction product; and (ii) a second step of adding dimethylformamide (DMF) to the reaction product of the first step.

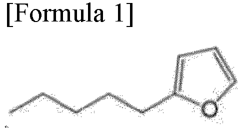

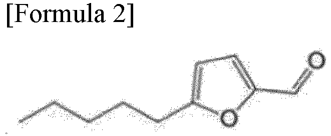

Another aspect of the present disclosure provides a 5-PF-amino acid (AA) derivative having a structure of the following Formula 3 or Formula 4; a hydrate or solvate thereof; or a pharmaceutically acceptable salt thereof.

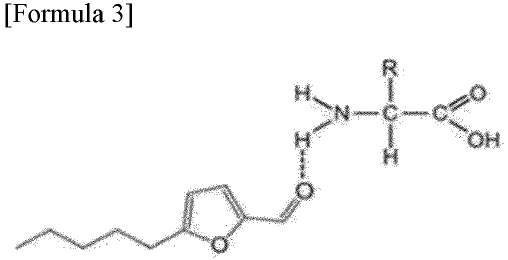

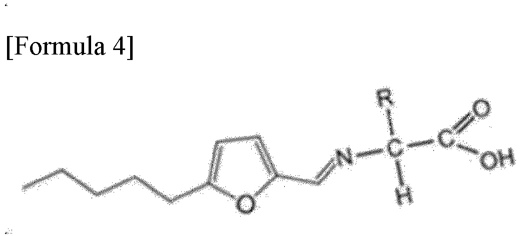

According to one embodiment of the present disclosure, in the formulas above, R is the same as defined in the [Best Mode for Carrying out the Invention] described below.

Still another aspect of the present disclosure provides a pharmaceutical composition for inhibiting adipocyte differentiation or treating metabolic diseases, comprising 5-PF or a 5-PF-amino acid (AA) derivative; a hydrate or solvate thereof; or a pharmaceutically acceptable salt thereof as an active ingredient.

Still another aspect of the present disclosure provides a health functional food for improving metabolic diseases, comprising 5-PF or a 5-PF-amino acid (AA) derivative; a hydrate or solvate thereof; or a pharmaceutically acceptable salt thereof as an active ingredient.

Still another aspect of the present disclosure provides a method for treating or preventing a metabolic disease in an individual, comprising administering 5-PF or a 5-PF-amino acid (AA) derivative; a hydrate or solvate thereof; or a pharmaceutically acceptable salt thereof.

Still another aspect of the present disclosure provides a use of 5-PF or a 5-PF-amino acid (AA) derivative; a hydrate or solvate thereof; or a pharmaceutically acceptable salt thereof for inhibiting adipocyte differentiation or treating or preventing metabolic diseases.

Still another aspect of the present disclosure provides a use for the preparation of a medicament for inhibiting adipocyte differentiation or treating metabolic diseases, comprising 5-PF or a 5-PF-amino acid (AA) derivative; a hydrate or solvate thereof; or a pharmaceutically acceptable salt thereof.

### Advantageous Effects of Invention

The pharmaceutical composition comprising 5-PF or a 5-PF-AA derivative as an active ingredient according to the present disclosure comprising 5-PF derived from natural products, and thus it is less likely to cause side effects because it is non-cytotoxic; additionally, since it has excellent inhibitory effects against adipocyte differentiation and inflammation, it can be very effectively used for improving, preventing, or treating metabolic diseases, such as obesity, adult disease, diabetes, obesity-type diabetes, vascular diabetic complications, and non-alcoholic steatohepatitis (NASH).

Additionally, the present disclosure exhibits functionality equivalent to or greater than that of existing drug doses even at lower doses compared to existing drug doses, it can be effectively used to improve, prevent, or treat metabolic diseases.

### Brief Description of Drawings

FIG. 1 shows the results of a comparative analysis via LC/MS between 5-PF (a) obtained from the extract of *Ceriporia lacerata* and 5-PF (b) obtained synthetically.
FIG. 2 shows the results of a comparative analysis via ¹H-NMR between 5-PF (a) obtained from the extract of *Ceriporia lacerata* and 5-PF (b) obtained synthetically, and the ¹³C-NMR result of the extracted 5-PF (c).
FIG. 3 shows the GC/MS analysis results of synthesized 5-PF.
FIG. 4 shows the measurement results of cell activity when 5-PF-glutamine(Q) (5-PF-Q), which is one of the 5-PF-AA derivative groups, was treated to (a) 3T3-L1 adipocytes, (b) C2C12 muscle cells, (c) HUVEC human vascular epithelial cells, and (d) HACAT human skin cells.
FIG 5 shows the results confirming the inhibitory effect of 5-PF-Q against adipocyte differentiation.
FIG. 6 shows the results confirming the effect of 5-PF-Q on the expression of adiponectin.
FIG. 7 shows the results confirming the effect of 5-PF-Q on the expression of AMPK.
FIG. 8 shows the results confirming the effect of 5-PF-Q on the expression of inflammatory proteins and cytokines.
FIG. 9 shows the results confirming the inhibitory effect of 5-PF-Q against fatty liver or steatosis through inhibition of adipocyte differentiation.
FIG. 10 shows the results confirming the effects of 5-PF-Q on glycated hemoglobin and insulin.
FIG. 11 shows the results confirming the effects of 5-PF-Q on TC, TG, ALP, AST, and ALT.
FIGS. 12 and 13 show the results of confirming the changes in body weight and food intake over 6 weeks when 5-PF-Q was administered to Ob/Ob mice groups A and B, respectively.
FIGS. 14 and 15 show the results of confirming the reduction in the amount of blood sugar when performing an OGTT test after administering 5-PF-Q to Ob/Ob mice groups A and group B, respectively.
FIG. 16 shows the results of confirming the changes in liver indexes (ALT, AST, ALP, LDH), kidney indexes (CREA, BUN), and lipid indexes (cholesterol, TG, LDL, HDL, LDH) in the Ob/Ob mice groups treated with 5-PF-Q.
FIG. 17 shows the results of confirming whether insulin resistance was improved in the Ob/Ob mice groups treated with 5-PF-Q.

### Best Mode for Carrying out the Invention

Hereinafter, various implementation examples are described in detail so that those with ordinary skill in the art to which the present disclosure belongs can easily implement the present disclosure. However, the implementation examples may be implemented in various different forms and are not limited to the implementation examples described in this specification.

In this specification, when a part is said to "comprise" a certain component, this does not mean that other components are excluded, but rather that other components may be included, unless otherwise specifically stated.

In this specification, singular expressions are interpreted to include the singular or plural as interpreted by the context, unless otherwise specified.

Additionally, all numbers and expressions indicating the amounts of ingredients, reaction conditions, *etc.* described in this specification should be understood as being modified by the term "about" in all cases unless otherwise specified.

In this specification, the terms first, second, *etc.* are used to describe various components, and the components should not be limited by the terms. These terms are used only for the purpose of distinguishing one component from another.

An aspect of the present disclosure provides a method for preparing a compound comprising a 5-pentylfurfural (5-PF) structure of the following Formula 2, which comprises (i) a first step of adding butyllithium (BuLi) dissolved in hexane to a compound of the following Formula 1 dissolved in an aprotic organic solvent to prepare a reaction product; and (ii) a second step of adding dimethylformamide (DMF) to the reaction product of the first step.

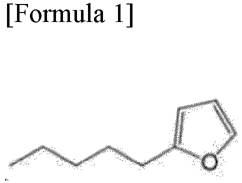

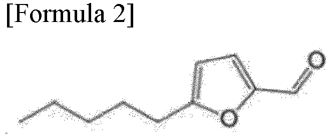

The compounds comprising the 5-PF structure above include a 5-PF-amino acid (AA) derivative, a hydrate or solvate thereof, or a pharmaceutically acceptable salt thereof.

In one embodiment of the present disclosure, the compound comprising the 5-PF structure may be a compound of the following Formula 2.

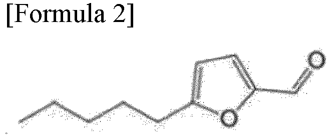

In one embodiment of the present disclosure, the aprotic organic solvent may be tetrahydrofuran (THF).

The molar concentration ratio of BuLi, which is added in the first step, and DMF, which is added in the second step, may be 1:1 to 1:2. Specifically, the molar concentration ratio of BuLi, which is added in the first step, and DMF, which is added in the second step, may be 1:1 to 1:1.8, 1:1 to 1:1.6, 1:1 to 1:1.5*,* or 1:1 to 1:1.4, but is not limited to these ratios.

In one embodiment of the present disclosure, the first and/or second steps may be performed at a temperature of -10°C to 10°C. Specifically, the first step may be performed at a temperature of -8°C to 8°C, -7°C to 7°C, -6°C to 6°C, -5°C to 5°C, -4°C to 4°C, -3°C to 3°C, -2°C to 2°C, or -1°C to 1°C, but the temperature is not limited to these values.

In one embodiment of the present disclosure, the method may further comprise a step of stirring after the first step; and/or a step of stirring after the second step.

In one embodiment of the present disclosure, the method may further comprise a step of extracting with methyl tertiary butyl ether (MTBE) and a step of washing with brine after the second step.

Another aspect of the present disclosure provides a 5-PF-amino acid (AA) derivative having the structure of the following Formula 3; a hydrate or solvate thereof; or a pharmaceutically acceptable salt thereof:

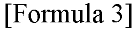

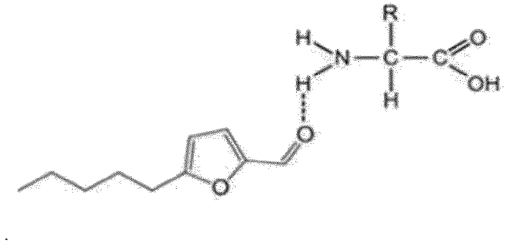

In Formula 3 above, R may be selected from the group consisting of 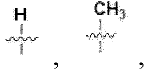 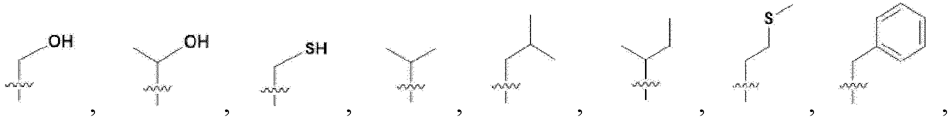 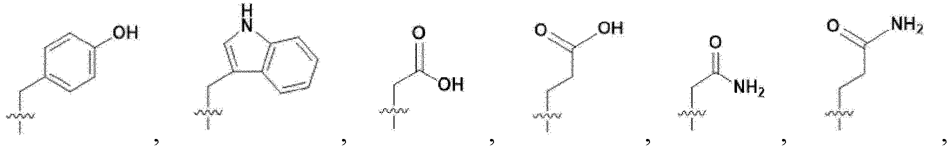 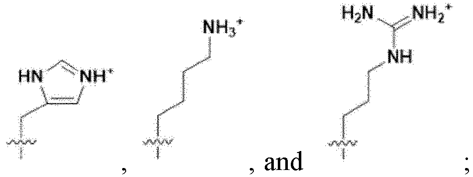 or R may bind to a nitrogen atom adjacent to a carbon atom, to which R is attached, to form a pyrrolidine ring.

Still another aspect of the present disclosure provides a 5-PF-amino acid (AA) derivative having a structure of the following Formula 4; a hydrate or solvate thereof; or a pharmaceutically acceptable salt thereof:

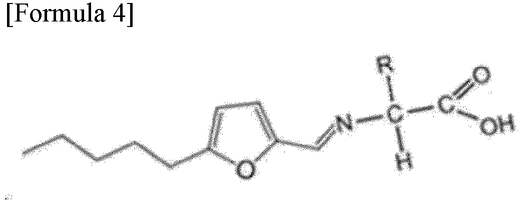

In Formula 4 above, R may be selected from the group consisting of 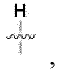 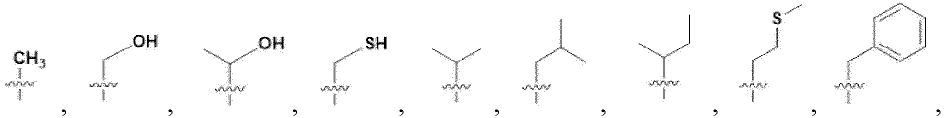 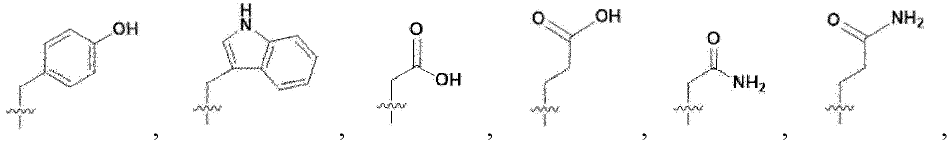 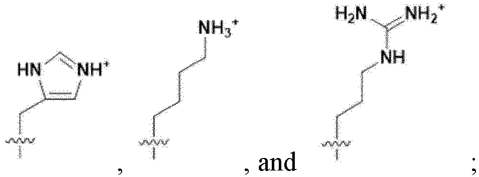 or R may bind to a nitrogen atom adjacent to a carbon atom, to which R is attached, to form a pyrrolidine ring.

In one embodiment of the present disclosure, the structural formula of Formula 4, where R binds to a nitrogen atom adjacent to a carbon atom to which it is attached to form a pyrrolidine ring, is the same as the following Formula 5 shown below.

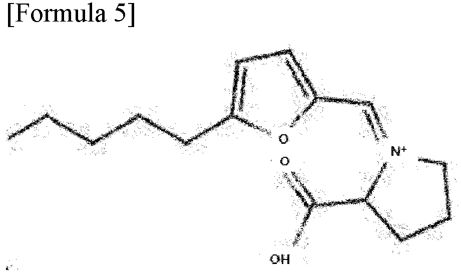

In one embodiment of the present disclosure, in the compound of Formula 3 above, R may be 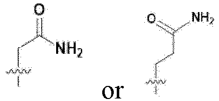 , but is not limited thereto.

In one embodiment of the present disclosure, in the compound of Formula 4 above, R may be 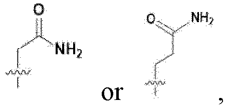 but is not limited thereto.

The 5-PF or a 5-PF-amino acid (AA) derivative may inhibit the activity of at least one of α-amylase, α-glucosidase, and lipase. α-Amylase or α-glucosidase are carbohydrate-decomposing enzymes, and lipase is a fat-decomposing enzyme. Medicaments that can inhibit the activity of these enzymes have been developed to control blood sugar levels and improve lipid metabolism in patients with metabolic diseases such as diabetes or obesity (*e.g*., Orlistat); however, due to various side effects and difficulty in synthesis, there has been a demand for the development of natural products with similar activities. The pharmaceutical composition of the present disclosure comprising the 5-PF or a 5-PF-amino acid (AA) derivative as an active ingredient may be usefully used for the prevention, treatment, and improvement of diabetes by directly inhibiting the decomposition of fat.

The 5-PF or a 5-PF-amino acid (AA) derivative may exhibit the effect of inhibiting the expression of at least one among CCAAT-enhancer-binding protein α (C/EBPα), CCAAT-enhancer-binding protein β (C/EBPβ), peroxisome proliferator activated receptory (PPARγ), adiponectin, and fatty acid binding protein 4 (FABP4), thereby increasing the expression of the glucose transporter type 4 (GLUT4) gene.

In particular, adipocyte differentiation is a complex process involving changes in morphology, hormone sensitivity, and gene expression. Some of transcription factors have been shown to play important roles in adipocyte differentiation. The major transcription factors involved in the formation of adipocytes include C/EBPα and PPARγ. In particular, PPARγ increases the expression of adipogenesis marker genes, such as adiponectin and FABP4. In addition, GLUT4 is a glucose transporter gene, and when it is stimulated by insulin, GLUT4 within the cell moves to the plasma membrane to promote glucose transport. Therefore, GLUT4 plays a role in lowering blood sugar levels by facilitating the influx of blood glucose into cells. That is, the antidiabetic effect increases with the increase of the activity and expression of GLUT4, which is involved in glucose transport.

Still another aspect of the present disclosure provides a pharmaceutical composition for inhibiting adipocyte differentiation or treating metabolic diseases, comprising 5-PF or a 5-PF-amino acid (AA) derivative; a hydrate or solvate thereof; or a pharmaceutically acceptable salt thereof as an active ingredient.

The pharmaceutically acceptable salt of the 5-PF or a 5-PF-amino acid (AA) derivative thereof has low toxicity to the human body and does not inhibit the biological activity and physicochemical properties of 5-PF or 5-PF-amino acid (AA). In an embodiment, the pharmaceutically acceptable salt includes, acid addition salts of the pharmaceutically usable free acid and 5-PF or 5-PF-AA, alkali metal salts (*e.g.*, sodium salts, *etc.*) and alkaline earth metal salts (*e.g.*, calcium salts, *etc.*), but is not limited thereto.

In one embodiment of the present disclosure, the acid addition salt may be a salt with an inorganic acid or organic acid. As the inorganic acid, hydrochloric acid, sulfuric acid, nitric acid, phosphoric acid, perchloric acid, bromic acid, *etc.* may be used. Additionally, as the organic acid, acetic acid, methanesulfonic acid, ethanesulfonic acid, *p*-toluenesulfonic acid, fumaric acid, maleic acid, malonic acid, phthalic acid, succinic acid, lactic acid, citric acid, citric acid, gluconic acid, tartaric acid, salicylic acid, malic acid, oxalic acid, benzoic acid, embonic acid, aspartic acid, glutamic acid, *etc.* may be used. These salts may be prepared by conventional methods. For example, these salts may be prepared by dissolving the 5-PF or 5-PF-AA in a solvent that can be mixed with water (*e.g.*, methanol, ethanol, acetone, and 1,4-dioxane), and adding a free acid or a free base followed by crystallization of the resultant.

In one embodiment of the present disclosure, a hydrate or solvate of the 5-PF or 5-PF-amino acid (AA) may be prepared by a known method, and it is preferable that the hydrate or solvate be non-toxic and water-soluble, and it is preferable that the compound be a hydrate or solvate in which 1 to 5 molecules of water or an alcohol solvent (especially, ethanol, *etc.*) are linked.

In one embodiment of the present disclosure, the pharmaceutical composition for inhibiting adipocyte differentiation or treating metabolic diseases may further comprise one or more known substances having the effect of improving metabolic disease- (anti-obesity/anti-diabetic effects, *etc.*), in addition to 5-PF or 5-PF-amino acid (AA). The known substances may be an inhibitor of C/EBPα, C/EBPβ, PPARγ, adiponectin, or FABP4; or a promoter of GLUT4, but are not limited thereto.

In one embodiment of the present disclosure, the pharmaceutical composition for inhibiting adipocyte differentiation or treating metabolic diseases may further comprise one or more pharmaceutically acceptable additives selected from the group consisting of excipients, lubricants, wetting agents, sweeteners, flavoring agents and preservatives.

In one embodiment of the present disclosure, the pharmaceutical composition for inhibiting adipocyte differentiation or treating metabolic diseases may be formulated according to conventional methods to be used. In particular, the pharmaceutical composition may be formulated by adopting methods known in the art so as to provide rapid, sustained, or delayed release of the active ingredients after administration to mammals.

In one embodiment of the present disclosure, the method for administering the pharmaceutical composition for inhibiting adipocyte differentiation or treating metabolic diseases may easily be selected depending on the formulation, and may be administered orally or parenterally. For example, the pharmaceutical composition may be used via intradermal, intramuscular, intraperitoneal, intravenous, subcutaneous, intranasal, epidural, and oral routes, but the administration routes are not limited thereto. Solid formulations for oral administration may include tablets, pills, soft or hard capsules, pills, powders, and granules, but are not limited thereto. Meanwhile, formulation types for parenteral administration may include creams, lotions, ointments, pastes, liquids, patches, and injections, but are not limited thereto.

In one embodiment of the present disclosure, the dose of the pharmaceutical composition for inhibiting adipocyte differentiation or treating metabolic diseases may vary depending on the patient's age, sex, body weight, severity of disease, and administration route, but generally, the dose may be administered once to three times daily in divided doses of 0.5 mg/kg to 100 mg/kg, 0.5 mg/kg to 50 mg/kg, 0.5 mg/kg to 10 mg/kg, or 0.5 mg/kg to 5 mg/kg. However, the dose does not limit the scope of the present disclosure in any way.

In one embodiment of the present disclosure, the dose of the 5-PF or 5-PF-amino acid (AA) derivative; a hydrate or solvate thereof; or a pharmaceutically acceptable salt thereof may be 10 µg/mL or more, 20 µg/mL or more, 30 µg/mL or more, 40 µg/mL or more, or 50 µg/mL or more. Specifically, the dose may be 10 µg/mL to 140 µg/mL, 20 µg/mL to 130 µg/mL, 30 µg/mL to 120 µg/mL, 40 µg/mL to 110 µg/mL, or 50 µg/mL to 100 µg/mL, but is not limited thereto.

In one embodiment of the present disclosure, the metabolic disease may be any one selected from the group consisting of obesity, adult disease, diabetes, obesity-type diabetes, vascular diabetic complications, non-alcoholic steatohepatitis (NASH), dyslipidemia, arteriosclerosis, hyperlipidemia, hypercholesterolemia, and cardiovascular disease.

Still another aspect of the present disclosure provides a health functional food for improving metabolic diseases, comprising 5-PF or a 5-PF-amino acid (AA) derivative; a hydrate or solvate thereof; or a pharmaceutically acceptable salt thereof as an active ingredient.

In one embodiment of the present disclosure, the health functional food may comprising the 5-PF or 5-PF-amino acid (AA); a hydrate or solvate thereof; and/or a pharmaceutically acceptable salt thereof in an amount of 0.001 wt% to 100 wt%, 0.001 wt% to 50 wt%, 0.1 wt% to 100 wt%, 0.1 wt% to 50 wt%, or 0.1 wt% to 40 wt%, based on the total weight of the health functional food.

In one embodiment of the present disclosure, the health functional food may be in the form of powders, granules, tablets, capsules, or beverages, and may further comprise other natural or synthetic substances for health/function and additives, *etc.* for productization.

In one embodiment of the present disclosure, when the health functional food is a beverage, the 5-PF or 5-PF-amino acid (AA) and/or a pharmaceutically acceptable salt thereof may be included in an amount of 0.001 g to 2 g, 0.001 g to 1 g, 0.01 g to 2 g, 0.01 g to 1 g, or 0.01 g to 0.1 g, based on 100 mL of the health functional food. In addition, there are no particular restrictions on liquid ingredients, and various flavoring agents, natural carbohydrates, *etc.* may be included as additional ingredients in normal amounts, as in conventional beverages.

Examples of the above-mentioned natural carbohydrates may include monosaccharides; disaccharides such as glucose and fructose; polysaccharides such as maltose and sucrose; oligosaccharides such as dextrin and cyclodextrin; and sugar alcohols such as xylitol, sorbitol, and erythritol. As the above-mentioned flavoring agents, natural flavoring agents (thaumatin, stevia extracts (*e.g*., rebaudioside A, glycyrrhizin, *etc.*)), synthetic flavoring agents (saccharin, aspartame, *etc.*) may be used.

In addition, the health functional food for improving obesity/diabetes in the present disclosure may include various nutritional supplements, vitamins, minerals (electrolytes), flavoring agents including synthetic flavoring agents and natural flavoring agents, coloring agents and thickening agents (cheese, chocolate, *etc.*), pectic acid and salts thereof, alginic acid and salts thereof, organic acids, protective colloidal thickeners, pH regulators, stabilizers, preservatives, glycerin, alcohol, carbonating agents used in carbonated beverages, *etc.* In addition, the health functional food may include fruit pulp for the production of natural fruit juices, fruit juice drinks and vegetable drinks. These ingredients may be used independently or in combination.

The health functional food for improving metabolic diseases, comprising 5-PF or a 5-PF-amino acid (AA) derivative; a hydrate or solvate thereof; or a pharmaceutically acceptable salt thereof as an active ingredient, may include, for example, various foods, beverages, gums, vitamin complexes, health supplement foods, *etc.*

Still another aspect of the present disclosure provides a method for treating or preventing a metabolic disease in an individual, comprising administering 5-PF or a 5-PF-amino acid (AA) derivative; a hydrate or solvate thereof; or a pharmaceutically acceptable salt thereof.

Still another aspect of the present disclosure provides a use of 5-PF or a 5-PF-amino acid (AA) derivative; a hydrate or solvate thereof; or a pharmaceutically acceptable salt thereof for inhibiting adipocyte differentiation or treating or preventing metabolic diseases.

Still another aspect of the present disclosure provides a use for the preparation of a medicament for inhibiting adipocyte differentiation or treating or preventing metabolic diseases, comprising 5-PF or a 5-PF-amino acid (AA) derivative; a hydrate or solvate thereof; or a pharmaceutically acceptable salt thereof.

Hereinafter, the present disclosure will be described in more detail by the following Examples. However, the following Examples are only intended to illustrate the present disclosure, and the scope of the present disclosure is not limited to these Examples.

### [Example]

### Preparation Example 1. Method for organic synthesis of 5-pentylfurfural (5-PF) or 5-PF-AA

BuLi (1.1 eq) dissolved in hexane was added to 50 g of the following Compound 1 dissolved in 500 mL of THF at 0°C. After stirring for 30 minutes, DMF (1.5 eq) was added at the same temperature and the reaction mixture was stirred overnight. After stirring overnight, the resultant was poured into 1 M HCl in ice water, extracted with MTBE (200 mL × 3), washed with brine (200 mL × 3), dried over Na₂SO₄, and evaporated under vacuum. The concentrated crude product was distilled under reduced pressure (1 mm, 90°C) to obtain pure Compound 2 below. The amount synthesized was 31 g, and the yield was shown to be 95 to 99% by NMR analysis.

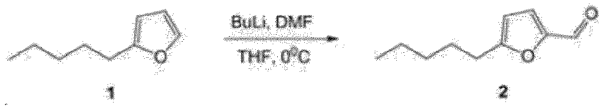

While HMF triflate was synthesized using the Grignard substitution method at an extremely low reaction temperature of -78°C in the existing synthesis method, in the present disclosure, the synthesis was performed at a relatively mild reaction environment of 0°C.

Tertiary amides can be readily used to form ketones and aldehydes because they react only once with Grignard and organolithium reagents, and in this Preparation Example, DMF was used to introduce a formyl group.

Additionally, a novel derivative may be generated by a one-pot reaction of 5-PF with an amino acid (AA) buffer (*e.g*., glutamine) including an amine group that may easily be linked to the aldehyde group of Compound 2. The aldehyde group of Compound 2 and the amine group of the amino acid sodium salt form a hydrogen bond between the keton (receptor) and the amide (donor) to thereby produce the following derivative Compound 3, or 5-PF-AA group, which is a novel derivative, may be obtained by forming the following derivative Compound 4 through a furfurylimine covalent bond, and 20 or more new derivatives can be obtained by combining 20 types of amino acids (Green Chem., 2015, 17, 4151-4156).

Specifically, as in Compound 4 below, 5-PF dissolved in ethanol was mixed with a buffer (pH 8.5) added with 10 mM L-glutamine (Q), which is one of the AA groups, at a molar ratio of 1:1, and then reacted at 37°C for 1 hour to obtain 5-PF-Q.

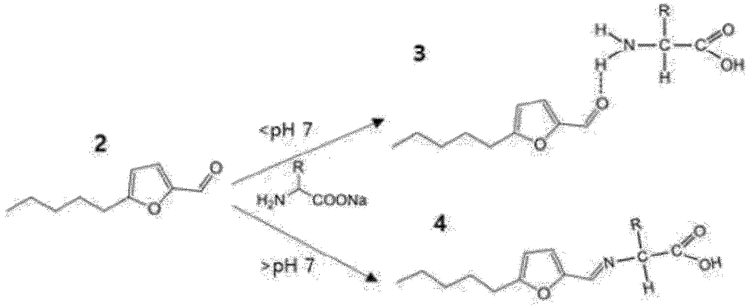

### Experimental Example 1. Method of 5-PF analysis

### 1-1: Comparative analysis of 5-PF compound obtained from Ceriporia extract and synthesized 5-PF compound through LC/MS

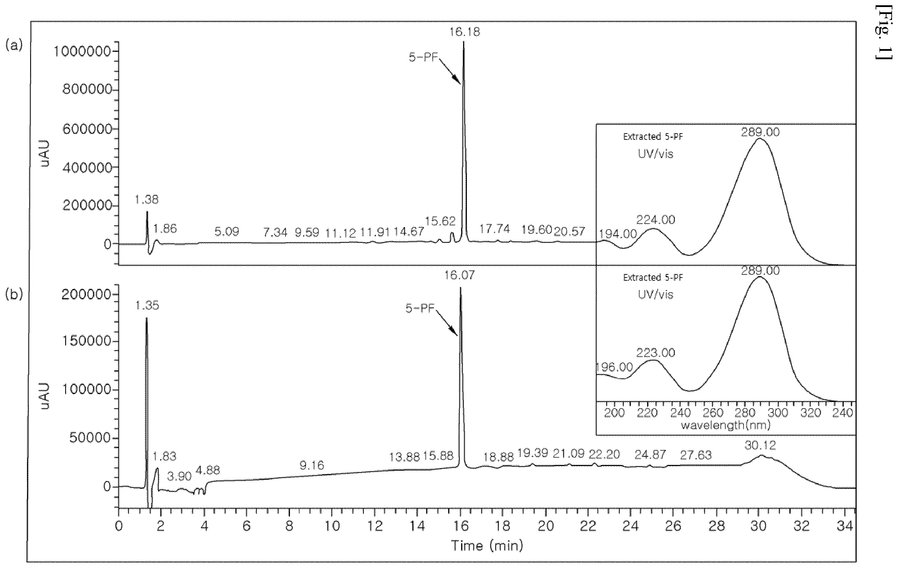

A sample (a) of the 5-PF compound obtained by ethyl acetate extraction of a mycelial culture of *Ceriporia lacerate* disclosed in Korean Patent No. 10-1031605 and a sample (b) of the 5-PF compound obtained through organic synthesis of Preparation Example 1 were compared and analyzed (FIG. 1).

Specifically, LC/MS was performed to compare the similarities between the extracted sample (a) and the synthesized sample (b). The bioactive compounds present in the *Ceriporia* mycelia culture were separated using the UHPLC analysis system. The analysis was performed using the Thermo Scientific Ultimate 3000 UHPLC system coupled with a tandem mass spectrometer (LCQ Fleet, Thermo Fisher Scientific, MA, USA) equipped with a SCINChrom C18G 100A column (100 mm × 2.1 mm, 1.8 µm, Scinco, Australia). Samples were eluted using a gradient system consisting of Solvent A (0.1% formic acid in water, v/v) and Solvent B (0.1% formic acid in acetonitrile, v/v). A gradient elution at a flow rate of 0.2 mL/min was started at 5% B, increased to 100% B in 20 min, and changed to 5% B in 35 min, and the total run time was 40 min including 5 min of column conditioning time. The injection volume was 2 µL and the column temperature was set at 40 °C. Electrospray ionization of the analyte was used in positive mode. The mass spectra were collected in full scan mode over the mass range of 100 Da to 1,000 Da. The MS/MS spectra were acquired at a collision energy of 25 ev. As a result of the separated peak analysis, 5-PF was measured at 16.18 min and 16.07 min (a slight tR difference occurred depending on the equipment conditions), and as a result of the comparison by UV-vis spectroscopy mass spectroscopy built into the Thermo Scientific Ultimate 3000 UHPLC system, it was confirmed that the compounds in samples (a) and (b) were identical.

### 1-2: Comparative analysis of 5-PF compound obtained from Ceriporia extract and synthesized 5-PF compound through ¹H-NMR

The *Ceriporia* extract and the compounds synthesized in Preparation Example 1 were compared by NMR spectroscopy. NMR data were obtained using the 700 MHz Bruker Avance III HD NMR spectrometer and a room temperature TBO probe. In order to correct for static magnetic field inhomogeneities, 3D and 1D shimming experiments were performed prior to NMR data collection, and data were obtained using a 1D nuclear Overhauser effect spectroscopy experiment with water suppression using 128 k points and 128 scans.

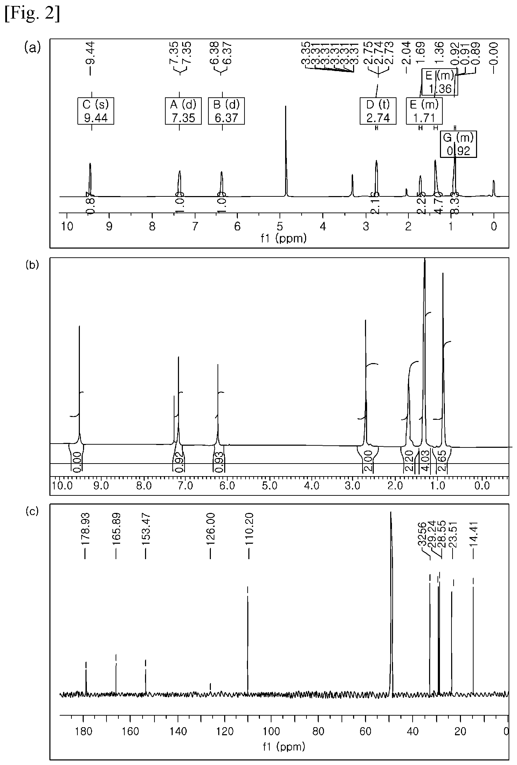

As a result of performing ¹H-NMR to compare the similarities between the extracted sample (a) and the synthesized sample (b), it was confirmed that the compounds of the samples (a) and (b) were identical (FIG. 2). In addition, the 5-PF structure was reconfirmed by the ¹³C-NMR results (c) of the synthesized sample.

### 1-3: GC/MS analysis of synthesized 5-PF compound

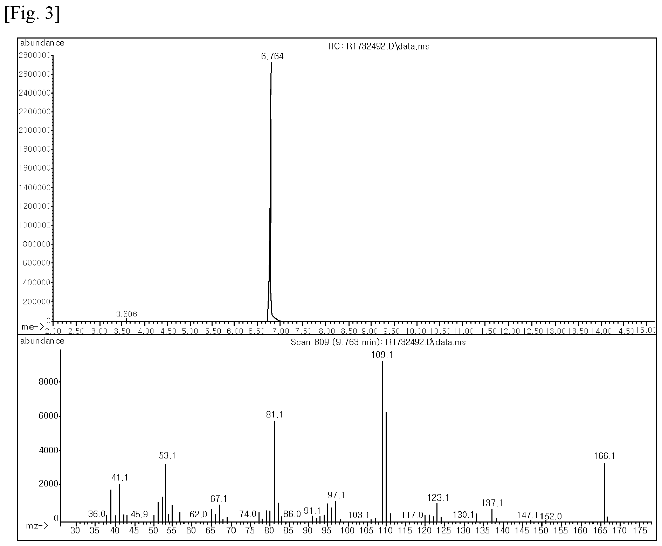

The compounds synthesized in Preparation Example 1 above were analyzed by GC/MS. The 5-PF samples were injected into the GC-MS injector equipped with an SPME manual holder and analyzed by HS-SPME-GC-MS (Agilent 7000C GC-MS system (Agilent Technologies, Wilmington, DE, USA)). The capillary column used for GC-MS analysis was a DB-5MS column (30 m × 0.25 mm I.D. × 0.25 µm, Agilent Technologies, Wilmington, DE, USA). The oven temperature was initially set at 60°C for 5 minutes and then increased by 3°C per minute to 250°C. The temperatures of the sample injector and detector were set at 250°C. The samples were injected using the splitless method. Helium (He, 99.999%) was used as the carrier gas, and the flow rate was 1.0 mL/min. The ionization energy was 70 eV, and the ions generated were analyzed by full-scan from m/z 30 to m/z 500. The analyte adsorbed on the SPME fiber was desorbed from the GC injector and injected into the GC capillary column for analysis. As a result, it was confirmed that the compound was a 5-PF molecule (FIG. 3).

### Example 1. Measurement of cytotoxicity/activity

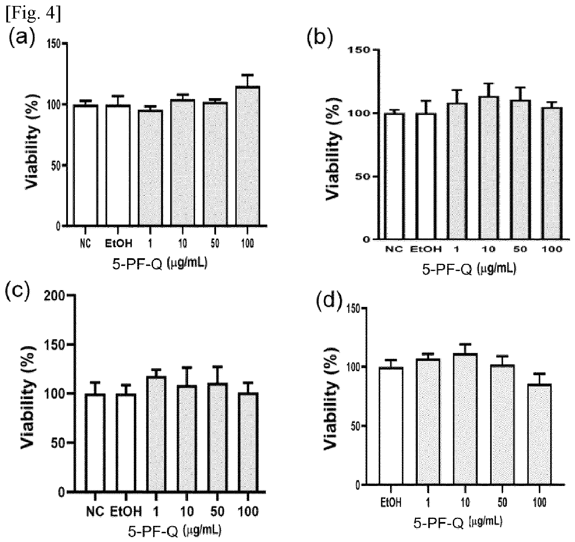

(a) 3T3-L1 Adipocytes (obtained from ATCC), (b) C2C12 muscle cells (obtained from ATCC), (c) HUVEC human vascular epithelial cells (obtained from ATCC), and (d) HACAT human skin cells (obtained from ATCC) were cultured at 1 × 10⁴ in Dulbeco's Modified Eagle's Media (DMEM, Gibco) high glucose medium (10% FBS, 1% penicillin/streptomycin), and the 5-PF-Q sample was added at various concentrations (1-100 µg/mL) and cultured for 24 hours. Then, the OD value at 450 nm was quantified using CCK8 solution and plotted (FIG. 4). A culture medium without a sample (NC) and a culture medium comprising 1% ethanol (EtOH) were used as negative controls. As shown in FIG 4, it was confirmed that when the 5-PF-Q sample was treated with various cell types, it did not affect cell activity and cytotoxicity.

### Example 2. Evaluation of effects of induction and inhibition of adipocyte differentiation

In the experiment to confirm the inhibition of adipogenesis, 3T3-L1 (adipocyte precursor cells) were differentiated into adipocytes in DMEM high glucose medium (including 10% calf serum, 1% penicillin/streptomycin) while simultaneously treating the samples. 3T3-L1 cells were cultured in a 24-well plate and differentiation was performed according to the flow chart below.

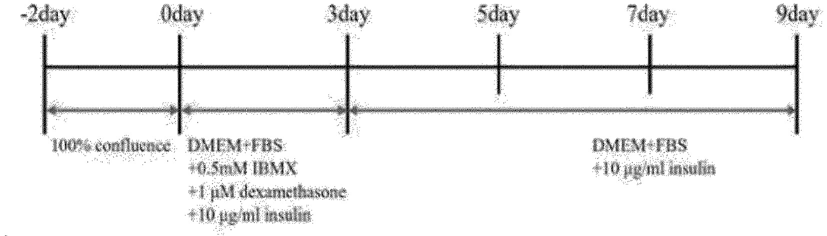

In order to confirm adipocyte differentiation, the 24-well plate was washed twice with PBS, and then the cells were fixed with 4% paraformaldehyde for 15 minutes. The cells were washed twice with PBS, and 60% isopropanol was added thereto, and the cells were left at room temperature for 5 minutes. After taking the 24-well plate out of the CO₂ incubator, the cell distribution was checked under an optical microscope (magnification: 40X) to confirm whether the confluency reached 100%, and then the medium was replaced with a fresh culture medium 1 mL/well. After 48 hours, the samples to be treated were diluted at various concentrations and replaced with 1 mL/well in a differentiation-inducing medium (which was prepared by mixing 10% FBS, 1% penicillin/streptomycin and an MDI solution (0.5 mM IBMX, 1 µM dexamethasone, 10 µg/mL insulin) with the DMEM high glucose medium). After 72 hours, the samples to be treated were diluted in the differentiation-inducting medium at various concentrations and replaced with 1 mL/well. Until Day 9 of differentiation, the samples to be treated in the differentiation-inducing medium were diluted at various concentrations and replaced with 1 ml/well every 48 hours. The 5-PF-Q was diluted at various concentrations and treated in the medium to be used from the starting day of differentiation to the completion day of differentiation (Day 9), and the concentrations used were 10 µg/mL, 50 µg/mL, and 100 µg/mL, and as the positive control, 0.5 mM metformin (90 µg/mL) was used.

For lipid staining, Oil red O solution was added thereto and the cells were stained at room temperature for 15 minutes, washed twice with PBS, and after microscopic observation, eluted with 100% isopropanol, and the absorbance was measured at 490 nm.

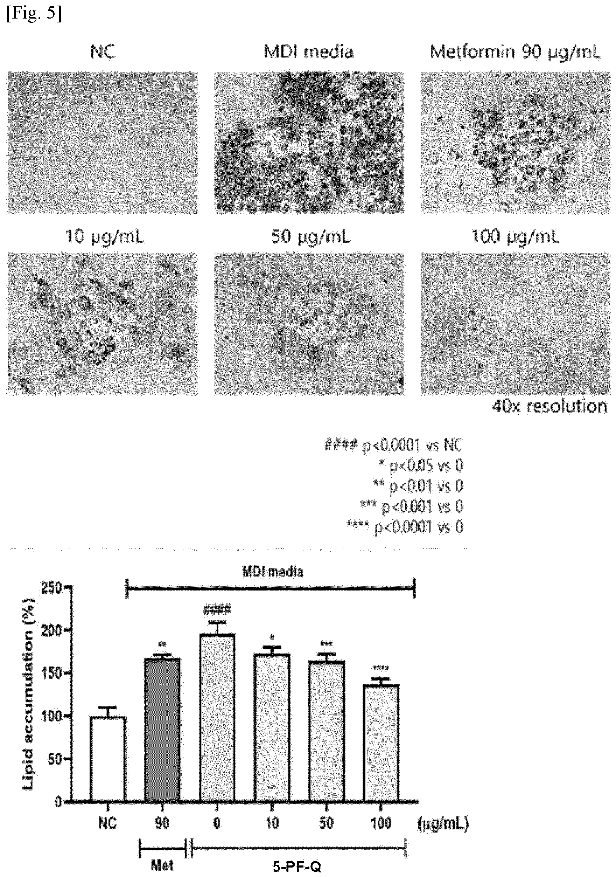

As a result of performing the experiment by treating the cells with 5-PF-Q at various concentrations while inducing adipogenesis for 9 days, adipogenesis was inhibited in a concentration-dependent manner in the group treated with the 5-PF-Q sample (FIG 5). From the comparison with the group treated with metformin 90 µg/mL (*i.e.,* the positive control group), it was found that the 5-PF-Q treatment group showed a tendency similar to metformin in the low concentration section (10 µg/mL), while showing a greater inhibition of adipogenesis than metformin in the high concentration section (50 µg/mL and 100 µg/mL).

### Example 3. Confirmation of effect on adiponectin expression

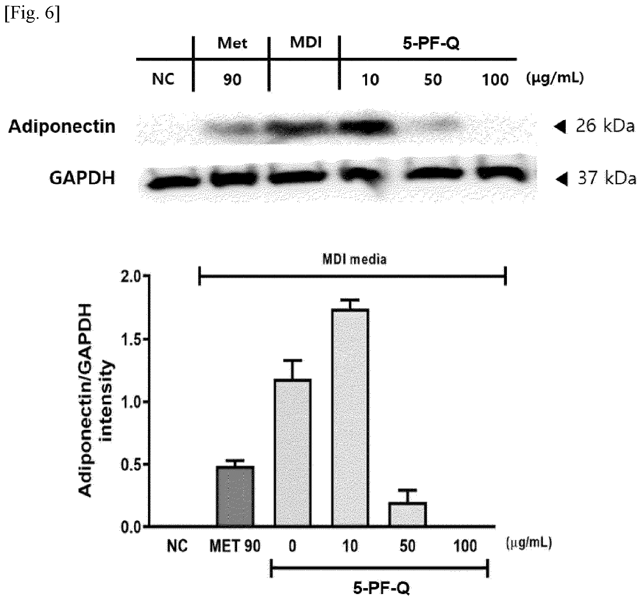

The effect of 5-PF-Q on the expression of adiponectin, which is a marker related to adipocyte differentiation, was confirmed through Western blotting (FIG. 6). Compared with the positive control group (*i.e.,* treatment with metformin at 90 µg/mL), the expression rate was 3.5-fold higher than that of metformin in the low concentration section (10 µg/mL), and the expression of adiponectin was clearly inhibited in the high concentration section (50 µg/mL and 100 µg/mL). It is speculated that peroxisome proliferator activated receptor γ (PPAR gamma), which is a master regulator of adiponectin expression, is regulated depending on the dose, and 5-PF-Q administration can inhibit adipocyte differentiation even in the low dose section (1 µg/mL to 40 µg/mL), while simultaneously modulating metabolism-related pharmacological functionality without toxicity or potential side effects in the high dose section (50 µg/mL to 100 µg/mL).

### Example 4. Confirmation of effect on AMPK expression

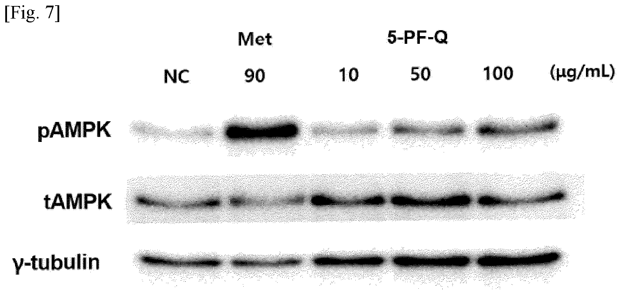

In order to confirm the effect of 5-PF-Q on the expression of AMP-activated protein kinase (AMPK), differentiated muscle cell C2C12 cells were treated with 5-PF-Q at 10 µg/mL, 50 µg/mL, and 100 µg/mL for 24 hours, respectively, as shown in FIG. 7 (10% gel, 20 µg PTN, 1° ab pAMPK 1:3,000, tAMPK 1:1,000/2° ab 1:4,000). The expression of AMPK was clearly observed in the positive control group (*i.e.,* treatment with metformin at 90 µg/mL), and it was confirmed that 5-PF-Q also activated the expression of AMPK in a concentration-dependent manner (FIG. 7).

### Example 5. Confirmation of effect on expression of inflammation-inducing proteins and cytokines

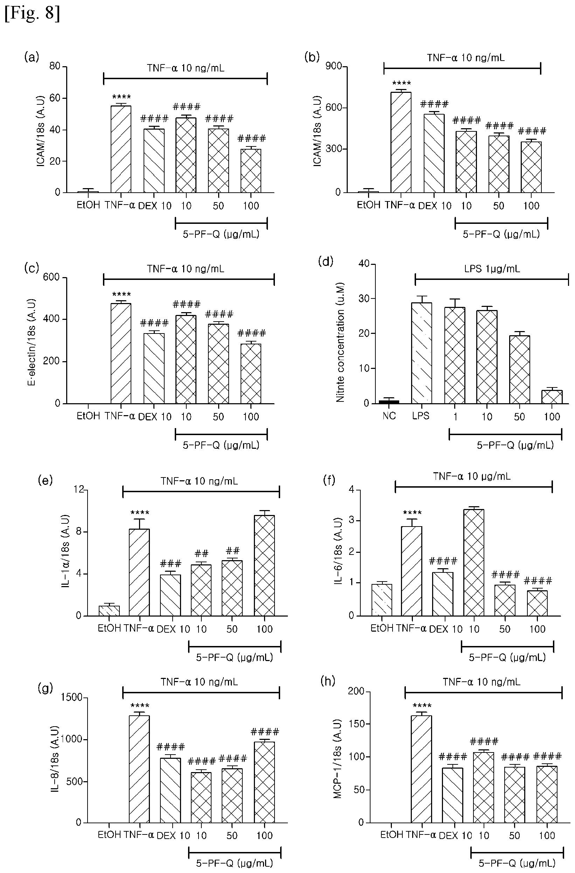

In order to determine the effect of 5-PF-Q on metabolic diseases such as diabetic complications that cause inflammation in blood vessels, the expression rate of inflammation-related cytokines was measured (FIG 8). HUVEC cells, which are vascular cells, were cultured, pretreated for 30 minutes, and then treated with TNF-α (10 ng/mL) for 4 hours. As a result of performing real-time PCR after RNA prep, the expression of increased adhesion molecules was decreased in a concentration-dependent manner in the group treated with TNF-α, and the expression level of intercellular adhesion molecule (ICAM) showed a slightly lower level of expression when treated with 5-PF-Q (50 µg/mL), and E-selectin showed a slightly lower level of expression when treated with 5-PF-Q (100 µg/mL), compared to dexamethasone, and particularly, vascular cell adhesion molecule (VCAM) showed a lower level of expression when treated with 5-PF-Q (10 µg/mL) compared to the treatment with dexamethasone.

In order to observe the ability of 5-PF-Q on inhibition of NO, RAW 264.7 cells were cultured at 5 ×10⁴ cells/well and then treated with samples of 5-PF-Q at various concentrations and LPS for 24 hours. The supernatants were obtained and a Griess reagent assay was performed, and the OD values were measured at 520 nm. The level of NO, which was increased by LPS, was decreased in a concentration-dependent manner in the group treated with 5-PF-Q. It was confirmed that the expression level was slightly decreased in the low concentration section (1-10 µg/mL), whereas the expression level was decreased rapidly in the high concentration section (50 µg/mL and 100 µg/mL).

The expression levels of IL-6 and monocyte chemotactic protein-1 (MCP-1) were decreased in a 5-PF-Q concentration-dependent manner, and the expression levels were shown to be similar to or lower than those treated with dexamethasone at 50 µg/mL and 100 µg/mL. On the other hand, the expression levels of IL-1α and IL-8 increased in a 5-PF-Q concentration-dependent manner, and showed expression levels similar to or lower than dexamethasone at 10 and 50 µg/mL.

### Example 6. Evaluation effect on inhibition of adipocyte differentiation in vivo and evaluation of biochemical impact

As shown in Table 1 below, histopathological results according to adipogenesis were obtained through *in vivo* experiments in which 5-PF-Q was administered to a group of diabetic mice.

**[Table 1]**

| Group | Sex | No. of Animals | Animal ID | Strain | Volume (mL/kg) | Dose (mg/kg) |
|---|---|---|---|---|---|---|
| G1 Normal | Male | 5 | 1101 to 1105 | db/m+ | 10 | 0 |
| G2 Control | | 5 | 1201 to 1205 | db/db | | 0 |
| G3 Positive (Met) | | 5 | 1301 to 1305 | | | 150 |
| G4 5-PF-Q | | 5 | 1401 to 1405 | | | 15 |

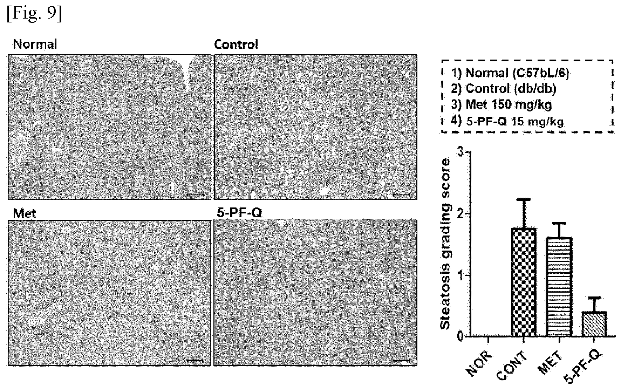

In a tissue examination performed after biopsy by sacrificing the mice, the percentage of hepatocytes with fatty deposition was scored as follows: less than 5% = 0; 5% to 33% = 1; 33% to 66% = 2; and 66% or more = 3, thus indicating that the 5-PF-Q administration group showed significantly lower fat deposition compared to the control group and the Met administration group (FIG 9). Through these results, it was confirmed that 5-PF-Q can have the effect of preventing or treating steatosis or steatohepatitis in the liver by inhibiting adipocyte differentiation.

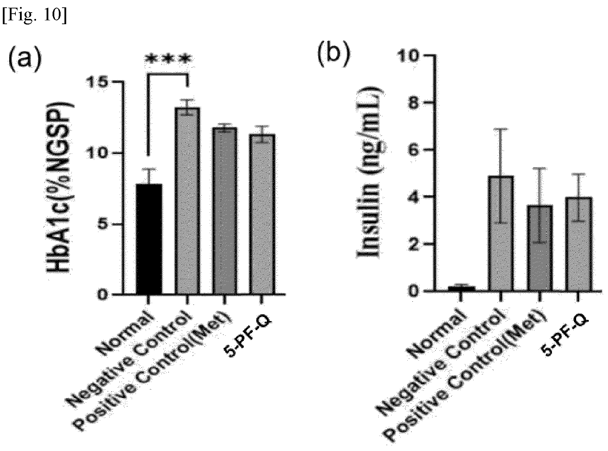

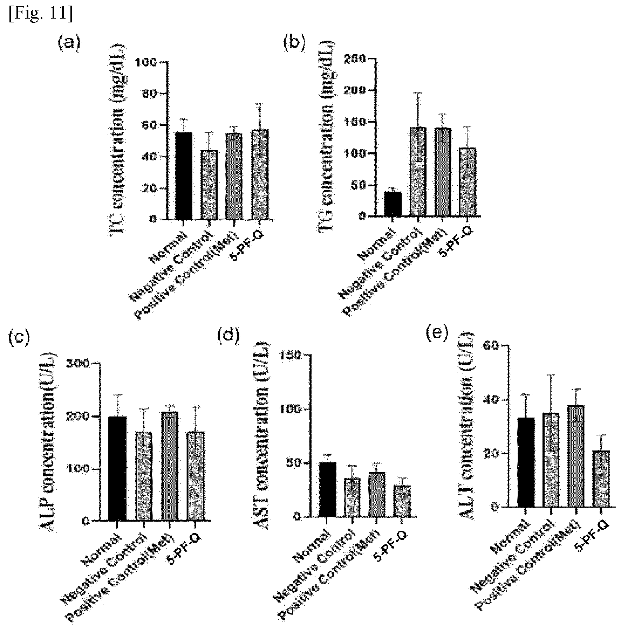

Additionally, blood biochemical test results revealed that 5-PF-Q has a tendency to reduce the levels of glycated hemoglobin and insulin (FIG. 10) and a tendency to reduce TG levels, but 5-PF-Q does not affect biochemical values such as TC, ALP, AST, and ALT (FIG 11). These results indicate that 5-PF-Q is not toxic to the liver.

### Example 7. Confirmation of antidiabetic effect in Ob/Ob mice

In order to confirm the antidiabetic effect of 5-PF-Q, the changes, due to the administration of 5-PF-Q, in body weight, food intake, reduction of blood sugar levels, toxicity, and insulin resistance were measured.

### 7-1: Measurement of mouse body weight and food intake

The mice were divided into group A (weight 35 g or more) and group B (weight 35 g or less) based on body weight. According to the flowchart below, each group was assigned to a metformin (200 mg/kg) administration group (positive control group) and a 5-PF-Q (15 mg/kg) administration group (experimental group) and administered orally for 3 weeks. After the 3-week administration period, blood sugar levels were measured by performing an oral glucose tolerance test (OGTT), and the mice were sacrificed 3 weeks thereafter. OGTT was also performed at the same time point for the Ob/Ob vehicle group (negative control group) that was not administered with the compound, and non-diabetic C57BL/6 mice, and they were sacrificed.

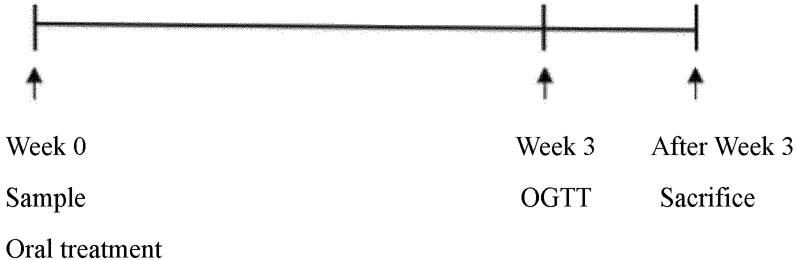

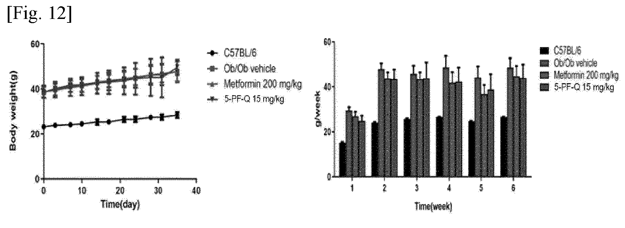

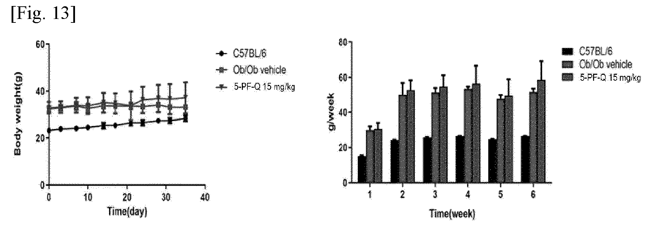

As a result, when body weight and food intake were checked once a week for 6 weeks in groups A and B, no significant difference in body weight or food intake was confirmed between the groups (FIGS. 12 and 13).

### 7-2: Measurement of blood glucose levels using mouse OGTT test

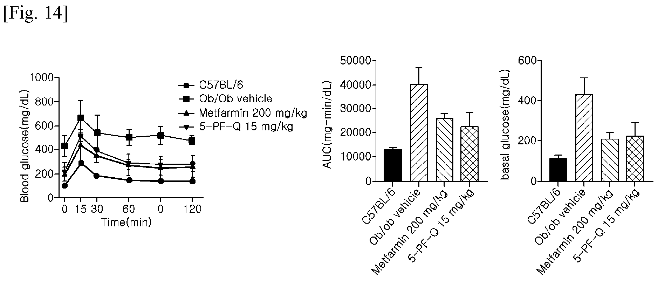

Upon reviewing the results of the OGTT performed on groups A and B classified in 7-1 above, it was found that while in group A, the blood sugar levels were decreased in a gentle curve in the Ob/Ob vehicle group (negative control group), the blood sugar reduction pattern was similar in the metformin administration group (positive control group) and the 5-PF-Q administration group (experimental group) (FIG. 14).

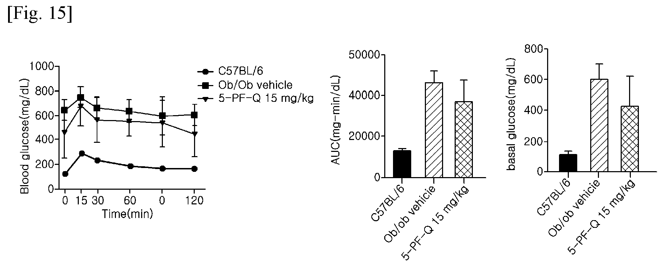

Similarly, in group B, the Ob/Ob vehicle group (negative control group) showed a gentle curve in the decrease of blood sugar levels, and the 5-PF-Q administration group (experimental group) showed a tendency for blood sugar levels to decrease more than the negative control group (FIG. 15).

### 7-3: Confirmation of toxicity in mouse liver

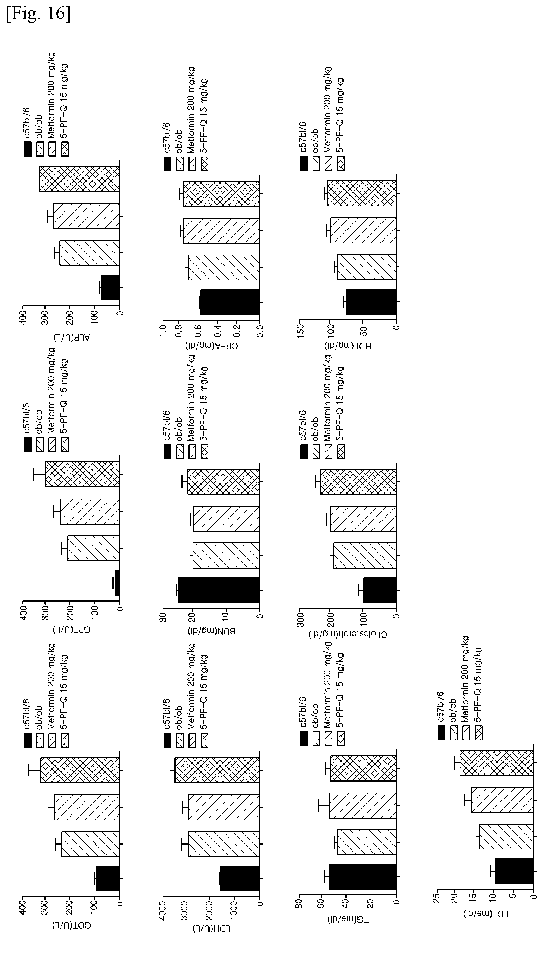

As a result of the blood test performed on the mice experimented with in 7-1 above, it was confirmed that there was no significant difference in ALT, AST, ALP, and LDH (*i.e.,* liver indexes), CREA and BUN (*i.e.,* kidney indexes), and cholesterol, TG, LDL, HDL, and LDH (*i.e.,* lipid indexes), in the 5-PF-Q administration group (experimental group). These results confirmed that 5-PF-Q is not toxic to the liver (FIG. 16).

### 7-4: Confirmation of insulin resistance in mice

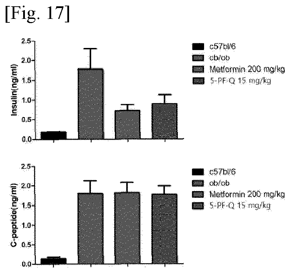

Compared to the Ob/Ob vehicle group (negative control group), both the 5-PF-Q administration group (experimental group) and the metformin administration group (positive control group) showed a tendency to improve insulin resistance, and in particular, the degree of improvement in insulin resistance was greater when 5-PF-Q was administered compared to metformin. Meanwhile, no significant difference was observed in C-peptide among all treatment groups. These results confirmed that 5-PF-Q helps improve insulin resistance (FIG. 17).

## Claims

1. A method for preparing a compound comprising a 5-pentylfurfural (5-PF) structure of the following Formula 2, comprising:
(i) a first step of adding butyllithium (BuLi) dissolved in hexane to a compound of the following Formula 1 dissolved in an aprotic organic solvent to prepare a reaction product; and
(ii) a second step of adding dimethylformamide (DMF) to the reaction product of the first step:

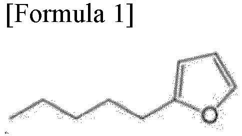

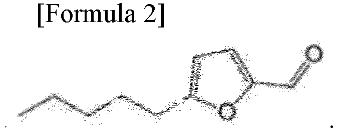

2. The method of claim 1, wherein the compound comprising the 5-PF structure is a compound of the following Formula 2:

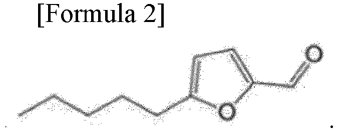

3. The method of claim 1, wherein the aprotic organic solvent is tetrahydrofuran (THF).

4. The method of claim 1, wherein the molar concentration ratio of BuLi added in the first step and DMF added in the second step is 1:1 to 1:2.

5. The method of claim 1, wherein the first and/or the second steps are performed at a temperature of -10°C to 10°C.

6. The method of claim 1, wherein the method further comprises stirring after the first step; and/or stirring after the second step.

7. The method of claim 1, wherein the method further comprises extracting with methyl tertiary butyl ether (MTBE) and washing with brine after the second step.

8. A 5-PF-amino acid (AA) derivative having a structure of the following Formula 3; a hydrate or solvate thereof; or a pharmaceutically acceptable salt thereof: 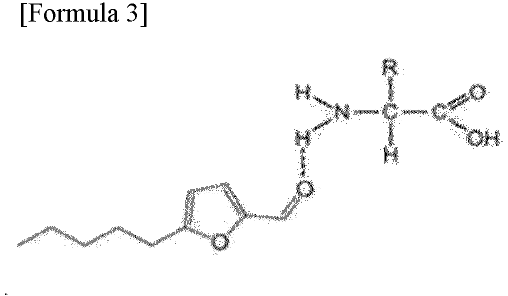 wherein in Formula 3 above, R is selected from the group consisting of 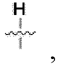 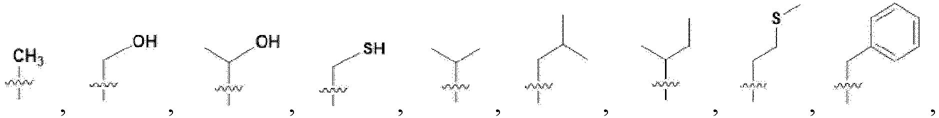 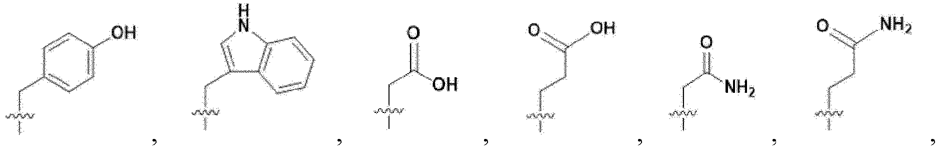 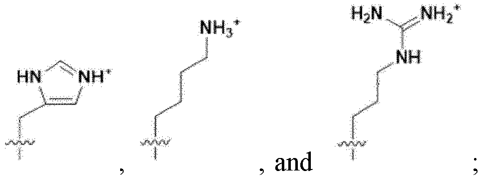 or R binds to a nitrogen atom adjacent to a carbon atom, to which R is attached, to form a pyrrolidine ring.

9. A 5-PF-amino acid (AA) derivative having a structure of the following Formula 4; a hydrate or solvate thereof; or a pharmaceutically acceptable salt thereof: 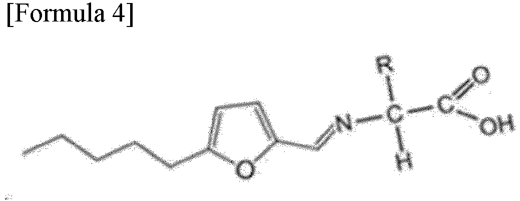 wherein in Formula 4 above, R is selected from the group consisting of 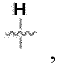 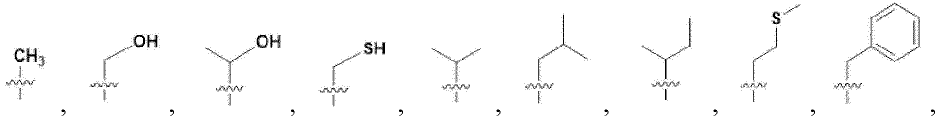 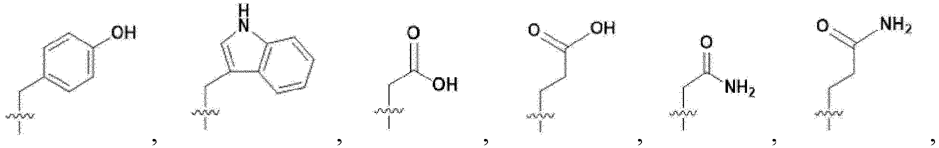 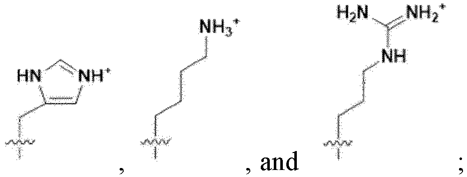 or R binds to a nitrogen atom adjacent to a carbon atom, to which R is attached, to form a pyrrolidine ring.

10. The 5-PF-amino acid (AA) derivative of claim 8; a hydrate or solvate thereof; or a pharmaceutically acceptable salt thereof,
wherein in Formula 3 above, R is

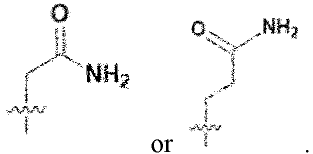

11. The 5-PF-amino acid (AA) derivative of claim 9; a hydrate or solvate thereof; or a pharmaceutically acceptable salt thereof,
wherein in Formula 4 above, R is

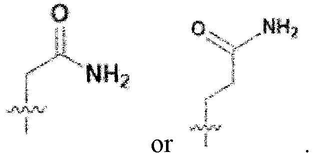

12. A pharmaceutical composition for inhibiting adipocyte differentiation or treating metabolic diseases, comprising 5-PF or a 5-PF-amino acid (AA) derivative; a hydrate or solvate thereof; or a pharmaceutically acceptable salt thereof as an active ingredient.

13. The pharmaceutical composition of claim 12, wherein the metabolic disease is any one selected from the group consisting of obesity, adult disease, diabetes, obesity-type diabetes, vascular diabetic complications, non-alcoholic steatohepatitis (NASH), dyslipidemia, arteriosclerosis, hyperlipidemia, hypercholesterolemia, and cardiovascular disease.

14. A health functional food for improving metabolic diseases, comprising 5-PF or a 5-PF-amino acid (AA) derivative; a hydrate or solvate thereof; or a pharmaceutically acceptable salt thereof as an active ingredient.

15. A method for treating or preventing a metabolic disease in an individual, comprising administering 5-PF or a 5-PF-amino acid (AA) derivative; a hydrate or solvate thereof; or a pharmaceutically acceptable salt thereof.

16. The method of claim 15, wherein the metabolic disease is any one selected from the group consisting of obesity, adult disease, diabetes, obesity-type diabetes, vascular diabetic complications, non-alcoholic steatohepatitis (NASH), dyslipidemia, arteriosclerosis, hyperlipidemia, hypercholesterolemia, and cardiovascular disease.

17. Use of 5-PF or a 5-PF-amino acid (AA) derivative; a hydrate or solvate thereof; or a pharmaceutically acceptable salt thereof for inhibiting adipocyte differentiation or treating or preventing metabolic diseases.

18. The use of claim 17, wherein the metabolic disease is any one selected from the group consisting of obesity, adult disease, diabetes, obesity-type diabetes, vascular diabetic complications, non-alcoholic steatohepatitis (NASH), dyslipidemia, arteriosclerosis, hyperlipidemia, hypercholesterolemia, and cardiovascular disease.

19. Use for the preparation of a medicament for inhibiting adipocyte differentiation or treating or preventing metabolic diseases, comprising 5-PF or a 5-PF-amino acid (AA) derivative; a hydrate or solvate thereof; or a pharmaceutically acceptable salt thereof.

20. The use of claim 19, wherein the metabolic disease is any one selected from the group consisting of obesity, adult disease, diabetes, obesity-type diabetes, vascular diabetic complications, non-alcoholic steatohepatitis (NASH), dyslipidemia, arteriosclerosis, hyperlipidemia, hypercholesterolemia, and cardiovascular disease.
